Europäisches Patentamt

European Patent Office

Office européen des brevets

(19)

(11) Veröffentlichungsnummer: **0 274 590 B1**

(12) **EUROPÄISCHE PATENTSCHRIFT**

(45) Veröffentlichungstag der Patentschrift: **10.07.91**

(21) Anmeldenummer: **87116262.4**

(22) Anmeldetag: **05.11.87**

(51) Int. Cl.⁵: **A61K 9/08**, A61K 31/35, A61K 47/00

(54) **Konservierte Augentropfen mit einem Gehalt an Cromoglycinsäure.**

(30) Priorität: **08.01.87 DE 3700379**

(43) Veröffentlichungstag der Anmeldung:
**20.07.88 Patentblatt 88/29**

(45) Bekanntmachung des Hinweises auf die
Patenterteilung:
**10.07.91 Patentblatt 91/28**

(84) Benannte Vertragsstaaten:
**AT BE CH ES FR GB LI LU NL**

(56) Entgegenhaltungen:
**EP-A- 0 036 351**
**AU-B- 543 418**
**FR-A- 2 139 872**

(73) Patentinhaber: **Dr. Gerhard Mann
chem.-pharm. Fabrik GmbH
Brunsbütteler Damm 165
W-1000 Berlin 20(DE)**

(72) Erfinder: **Bellmann, Günther, Dr., c/o Dr. Gerhard MannGmbH
Brunsbütteler Damm 165-173
W-1000 Berlin 20(DE)**

(74) Vertreter: **Siewers, Gescha, Dr.
Rechtsanwälte Dr. Harmsen, Dr. Utescher
Dipl.-Chem. Harmsen, Bartholatus Dr. Schaeffer, Dr. Fricke, Wolter Patentanwalt Dr. Siewers Adenauerallee 28
W-2000 Hamburg 1(DE)**

**Beschreibung**

Die Erfindung betrifft konservierte Augentropfen mit einem Gehalt an Cromoglicinsäure.

Cromoglicinsäure, nämlich 1,3-Bis-(2-carboxychromon-5-yloxy)-propan-2-ol ist eine bekannte Verbindung, die sowohl oral als auch topisch zur Behandlung von allergisch bedingten Erkrankungen wie beispielsweise Heufieber und damit verbundenen Konjunktividen und Rhinitiden eingesetzt wird. Aus der DE-PS 22 23 337 ist bereits eine wässrige Lösung zur Behandlung solcher Erkrankungen bekannt, die etwa 0,1 - 10 Gew.% des wasserlöslichen Salzes der Cromoglicinsäure zusammen mit 0,01 - 0,1 Gew.% EDTA und etwa 0,001 - 0,10 Gew.% eines Konservierungsmittels enthalten soll. Das nach der DE-PS 22 23 237 bevorzugt eingesetzte Konservierungsmittel ist Benzalkoniumchlorid, das eine Mischung von Alkylbenzyldimethylammoniumchloriden darstellt, bei denen die Alkylgruppe etwa 8 - 18 C Atome enthält. Die in dieser Vorveröffentlichung beschriebenen Augentropfen erfordern allerdings regelmäßig den Zusatz von EDTA, da Cromoglicinsäure mit geringen Metallspuren, wie sie aus pharmazeutischem Glas und den als Verschluß eingesetzten Gummi- oder Kunststoffstopfen herrühren, Niederschläge bildet, die für Augentropfen nicht annehmbar sind. Außerdem hat sich herausgestellt, daß auch die Cromoglicinsäure bzw. ihre Natriumsalze mit Benzalkoniumchlorid zu Niederschlägen führen, die sich allerdings in einem Überschuß der Cromoglicinsäure teilweise wieder auflösen; der Rest wird nach den Vorschriften der zitierten Patentschrift abfiltriert. Die vorbekannten Augentropfen sind im Hinblick hierauf äußerst unbefriedigend, da die Niederschlagsbildung zwischen Cromoglicinsäure und Benzalkoniumchlorid selbstverständlich für beide Verbindungen einen Wirkungsverlust bedeutet, der auch zu einer Ungenauigkeit in der Dosierung führt. Außerdem besteht in zwar relativ seltenen Fällen von prädisponierten Patienten der Wunsch, auf den Zusatz von EDTA bei der Behandlung allergischer Augenerkrankungen verzichten zu können, da EDTA, wenn auch nicht sehr stark ausgeprägt, selbst eine allergisierende Wirkung entfalten kann.

Der Erfindung liegt daher die Aufgabe zugrunde, Augentropfen auf der Basis von Cromoglicinsäure zu entwickeln, die die dargestellten Nachteile nicht aufweisen.

Zur Lösung der Aufgabe werden konservierte Augentropfen auf der Basis von Cromoglicinsäure oder ihren Salzen vorgeschlagen, die dadurch gekennzeichnet sind, daß sie Sorbit und Chlorbutanol enthalten.

Völlig überraschend wurde jetzt festgestellt, daß sich auch ohne Zusatz von EDTA völlig klare, temperaturbeständige und langfristig lagerfähige Augentropfen mit einem Gehalt an Cromoglicinsäure herstellen lassen, wenn man als Konservierungsmittel Chlorbutanol und als Isotonisierungsmittel Sorbit einsetzt.

Chlorbutanol ist an und für sich ein bekanntes Konservierungsmittel, das wegen seiner guten Verträglichkeit für Augentropfen geeignet ist und in der zitierten DE-PS 22 23 237 bereits in einer zahlreiche andere Verbindungen enthaltenden Aufzählung von Konservierungsmitteln erwähnt wird, allerdings immer nur unter der zusätzlichen Maßgabe, daß die Cromoglicinsäurelösung EDTA zur Verhinderung von Ausfällungen von Metallsalzen enthält. FR-A-2 139 872 beschreibt Augentropfen mit Cromoglicinsäure und EDTA, welche unteranderem zusätzlich Chlorbutanol enthalten. Sorbit ist als Zuckerabkömmling mit guter osmotischer Wirksamkeit zur Isotonisierung von Arzneimittellösungen geeignet und wird auch zur Isotonisierung von Augentropfen benutzt.

Wie umfangreiche Untersuchungen ergaben, führen in Cromoglicinsäurelösungen sehr geringe Metallspuren, die aus den Wandungen der Gefäße oder der Verschlüsse abgegeben werden, nach mehr oder weniger kurzer Lagerung zu Trübungen, die allerdings manchmal auch durch die zusätzlichen Konservierungsmittelmitbedingt sind. Die Lagerfähigkeit von pharmazeutischen Fertigpräparaten war daher bislang nur gering. Es war aber völlig überraschend, nunmehr festzustellen, daß Cromoglicinsäurelösungen ohne EDTA Zusatz vollständig stabil sind, wenn sie Chlorbutanol und Sorbit enthalten. Eine Erklärung für dieses außergewöhnliche Verhalten kann noch nicht gegeben werden, da Chlorbutanol keine komplexierenden Eigenschaften hat und dies für Sorbit nur in sehr geringem Maßstab zutrifft. Die Fähigkeit zur Bildung von Nebenvalenzbindungen oder Komplexen ist bei Sorbit im Gegensatz zu dem klassischen Sequestriermittel EDTA äußerst gering, so daß sich die Stabilität der erfindungsgemäß beanspruchten Lösungen bisher nicht erklären läßt. Dies gilt insbesondere in Anbetracht der Tatsache, daß die Erdalkali- und Nebengruppensalze der Cromoglicinsäure extrem schwerlöslich sind und daher einen relativ großen Zusatz an EDTA zur Stabilisierung benötigen.

Die erfindungsgemäßen Lösungen enthalten vorzugsweise bis 4% Cromoglicinsäure, 3-3,6% Sorbit und 0,5% Chlorbutanol, jeweils bezogen auf das Gewicht.

Die erfindungsgemäßen Augentropfen können in an sich bekannter Weise nach dem Stand der pharmazeutischen Technologie hergestellt werden, indem die Wirkstoffe in sterilem destilliertem Wasser gelöst und unter sterilen Bedingungen proportioniert und abgefüllt werden. Der Gehalt an Cromoglicinsäure bzw. ihrer wasserlöslichen Salze kann 1 - 5 Gew.%, derjenige an Sorbit ebenfalls 1 - 5 Gew.% und der an Chlorbutanol 0,1 - 0,8 Gew.% betragen.

Im folgenden wird die Erfindung anhand eines Beispieles näher erläutert:

1 Gew.% Cromoglicinsäure (in Form des Dinatriumsalzes) wird in etwa 2/3 des zur Herstellung benötigten sterilen destillierten Wassers aufgelöst. Diese Lösung wird dann mit 0,5% Chlorbutanol und 3,5% Sorbit versetzt. Nach vollständiger Lösung aller Verbindungen wird die Mischung mit destilliertem sterilem Wasser auf 100% aufgefüllt.

Die Herstellung erfolgtunter Laminar Flow - Bedingungen, ebenso wie die Konfektionierung.

## Ansprüche

1. Konservierte Augentropfen auf Basis Cromoglicinsäure, dadurch gekennzeichnet, daß sie Chlorbutanol und Sorbit enthalten.

2. Augentropfen nach Anspruch 1, dadurch gekennzeichnet, daß sie 1 bis 5 Gew.% Cromoglicinsäure oder ihrer pharmazeutisch verwendbaren wasserlöslichen Salze, 0,1 bis 0,8 Gew.% Chlorbutanol und 1 bis 5 Gew.% Sorbit enthalten.

## Claims

1. Preserved eyedrops on the basis of cromoglycate characterized in that they contain chlorobutanol and sorbite.

2. Eyedrops according to claim 1, characterized in that they contain 1 to 5 % by weight of cromoglycate or of its pharmaceutically usable water-soluble salts, 0,1 to 0,8 % by weight of chlorobutanol and 1 to 5 % by weight of sorbite.

## Revendications

1. Collyre liquide conservé à base de cromoglycate, caractérisé en ce qu'il contient du chlorobutanol et du sorbite.

2. Collyre liquide selon la revendication 1, caractérisé en ce qu'il contient de 1 à 5 % en poids de cromoglycate ou de ses sels solubles dans l'eau étant pharmaceutiquement utiles, de 0,1 à 0,8 % en poids de chlorobutanol et de 1 à 5 % en poids de sorbite.